# EUROPÄISCHE PATENTSCHRIFT

(11) **EP 2 620 176 B1**
(45) Veröffentlichungstag und Bekanntmachung des Hinweises auf die Patenterteilung: **17.01.2018**
(21) Anmeldenummer: 13401006.5
(22) Anmeldetag: 23.01.2013
(51) Int. Cl.: A61M 15/00, A61M 11/00

(54) **Inhalator mit atemgerecht angesteuertem Piezokristall**
Inhaler with breath-controlled piezo crystal
Inhalateur avec cristal piézoélectrique commandé par la respiration

(30) Priorität: 24.01.2012 DE 102012001342
(43) Veröffentlichungstag der Anmeldung: 31.07.2013
(73) Patentinhaber: NEBU-TEC GmbH, 63820 Elsenfeld (DE)
(72) Erfinder: Kern, Joachim, 63820 Elsenfeld (DE)
(74) Vertreter: Pöhner, Wilfried Anton

(56) Entgegenhaltungen:
- EP-A1- 0 923 957
- EP-A1- 2 216 100
- EP-A1- 2 221 079
- WO-A1-01/85241
- WO-A1-95/15822
- WO-A1-03/059424
- WO-A1-2009/042187
- US-A1- 2003 136 400
- US-A1- 2007 216 256

## Beschreibung

Die Erfindung bezieht sich auf einen Inhalator zum Inhalieren eines Wirkstoffes in Form eines Aerosols in die Lungen eines Lebewesens, gemäß Oberbegriff des Anspruches 1.

Das Inhalieren von Wirkstoffen, die in Wasser gelöst und als feiner Nebel eingeatmet werden, ist eine seit langem bekannte Methode zur Verabreichung von Wirkstoffen, insbesondere im medizinischen Bereich. Dabei hat sich das Prinzip der Wirkstoffbeförderung in die Lunge mittels eines Aerosols durch die mit Ultraschallverneblern möglichen, sehr kleinen Tröpfchen als besonders effizient herausgestellt. Es kann nachgewiesen werden, dass Tröpfchen unterhalb einer Mindestgröße vollständig in den Lungenblasen absorbiert werden und in das Blut transferiert werden.

Ein einfach aufgebauter Ultraschallvernebler ist aus der US 7,066,398 bekannt. Er besteht aus einem Behälter für die Wirkflüssigkeit, in dessen Unterseite eine Austrittsöffnung eingebracht ist. An der Unterseite des Wirkflüssigkeitsbehälters ist ein ringförmiger Piezokristall angebracht, der die Austrittsöffnung umschließt. Die Austrittsöffnung wird von einer Membran verschlossen, die auf der Unterseite des Piezokristalls aufliegt. Diese Membran ist von zahlreichen Poren durchbrochen.

Wenn an den Piezokristall eine Wechselspannung angelegt wird, deren Frequenz auf die mechanische Resonanzfrequenz des Piezokristalls abgestimmt ist, so gerät auch die damit verbundene Membrane in Schwingungen. Dadurch wird Wirkflüssigkeit durch die Poren der Membrane hindurch gedrückt und tritt auf der anderen Seite der Membran in Form sehr kleiner Flüssigkeitströpfchen wieder aus, die sich mit der umgebenden Luft zu einem Aerosol vermischen.

Die Membran schließt zugleich auch eine Öffnung ab, die entweder direkt in der Längsseite eines Atemrohres angeordnet ist oder die über einen Rohrstutzen damit verbunden ist, sodass ein T-förmiger Anschluss entsteht. Das Atemrohr ist mit der Lunge eines Lebewesens verbunden, wird also z.B. von einer Person an ihren Mund gehalten. Durch das Atemrohr hindurch wird Atemluft in die Lungen eingesaugt und wieder ausgestoßen.

Bei der praktischen Anwendung dieses Prinzips in einem möglichst einfachen Gerät ist die Dosierung der tatsächlich vom Patienten aufgenommenen Wirkstoffmenge abhängig vom Atemverhalten des Patienten, das bei den ganz einfachen Geräten weder überwacht noch in seinen Auswirkungen gesteuert wird.

Im Unterschied zur oralen Verabreichung - bei der auch ein unwilliger und/oder geschwächter und/oder unkonzentrierter Patient die Arzneimitteldosis entweder komplett aufnimmt oder gar nicht absorbiert, jedoch nur in einer praktisch vernachlässigbaren Anzahl von Fällen unbeabsichtigter Weise eine Teilmenge aufnimmt - wird also bei derart einfachen Inhalatoren häufig nur ein Teil der in ein Aerosol umgewandelten Wirkstoffmenge tatsächlich vom Patienten aufgenommen wird. Der übrige Teil wird entweder wieder vom Aerosolzustand in eine Flüssigkeit zurückverwandelt, oder lagert sich wegen unzureichender Luftgeschwindigkeit in den Rohrsystemen des Inhalators ab oder gelangt nur bis in den Mund- oder Rachenraum des Patienten.

Bei den einfachen Geräten ohne Zuluft- und Atemventil kann es auch vorkommen, dass der Patient das Aerosol nicht einatmet, sondern aus dem Gerät hinaus in die Umgebung bläst. Wenn der Patient kontinuierlich atmet und der Piezokristall kontinuierlich arbeitet, so werden während der Ausatmungs-Phase, die 2/3 der Gesamtzeit ausmacht, auch 2/3 der oft sehr hochwertigen und teuren Medikamente nicht genutzt sondern nach außen hin oder anderweitig abgegeben. Nur 1/3 der Medikamente gelangen in die Lunge.

Um dies zu vermeiden sind aufwendige Geräte bekannt, die bei Atmungsstillstand oder beim Ausatmen auch kein Aerosol erzeugen. Dazu sind sie mit Ventilen für Zuluft und Abluft sowie mit Messvorrichtungen für Fluss und/oder Druck ausgestattet sind. Zusätzlich werden auch noch Rohre und Leitungen benötigt, die diese Einrichtungen miteinander verbinden.

Der Nachteil dieser Geräte ist, dass der zusätzliche Aufwand das Gewicht und die Kosten deutlich erhöht. Das Risiko von Fehlfunktionen steigt. Alle zusätzlichen Rohre und anderen Bauelemente müssen regelmäßig und gründlich gereinigt werden. Widrigenfalls droht das Risiko zusätzlicher Keimbildung, die den Inhalator womöglich zu einer zusätzlichen Gesundheitsgefahr werden lassen können.

In der WO 01/85241 A1 wird ein Inhalator vorgeschlagen, bei dem die Erzeugung des Aerosols vermittels einer über an Piezokristall aktivierten Membran erfolgt und das Vorhandensein eines Aerosols und hieraus abgeleitet das Ein- und Ausatmen, mithilfe eines aus einer IR Sende- und Empfangseinheit bestehenden IR Sensors erfasst wird, der vom Aerosolerzeuger hinreichend beabstandet angebracht ist. Das Ein- und Ausatmen erfolgt in ein geschlossenes, in Abhängigkeit vom Betriebszustand mehr oder weniger mit Aerosol angereichertes Gefäßes.

Die WO 03/059424 A1 beschreibt Verfahren und Systeme zum Betreiben eines Aerosolgenerators, bei dem die Atemcharakteristik eines Patienten überwacht und zu vorteilhaften Zeitpunkten die Erzeugung und Einleitung des Aerosols in eine zum Patienten führendes Atemrohr gestartet und gestoppt wird. Die Einleitung erfolgt hierbei über eine Aerosolöffnung in einer Längsseite des Atemrohres.

Auf diesem Hintergrund hat sich die Erfindung die Aufgabe gestellt einen Inhalator aus einer möglichst geringen Anzahl an Bauteilen zur Verfügung zu stellen, der hierbei nichtsdestoweniger eine stabile und zuverlässige Funktion, d.h. Erkennung der Inhalationsphase, erlaubt.

Als Lösung lehrt die Erfindung, einen Inhalator mit den im Anspruch 1 angegebenen Merkmalen.

Das wichtiges Merkmal des erfindungsgemäßen Inhalators ist es also, den Piezokristall nicht nur als Aktor zu nutzen, der die Membran in Schwingungen versetzt, sondern den Piezokristall alternierend auch als Sensor zu verwenden, der die tatsächlichen Druckverhältnisse erfasst. Der Piezokristall und die darauf befestigte und aufgespannte Membrane wirken dann ähnlich wie ein Mikrofon. Für beide Vorgänge, also sowohl für das Antreiben der Membrane als Aktor zum Erzeugen von Schwingungen und damit Bildung von Aerosol als auch bei der Erfassung der Reaktion der Membrane auf die Druckänderungen werden ein und dieselben elektrischen Zuleitungen genutzt.
Aus der vom Piezokristall empfangenen Spannung kann die elektronische Steuerung auswerten, welcher Druck an der Membran herrscht. Wenn Unterdruck herrscht, wenn also eingeatmet wird, wird die Ansteuerung des Piezokristalls als Aktor aktiviert, so dass Aerosol erzeugt wird.

Diese Ansteuerung wird immer wieder unterbrochen, um zu prüfen, ob sich der Druck im Atemrohr geändert hat. Wenn nach wie vor ein Unterdruck herrscht, wird wieder zur Aerosolerzeugung umgeschaltet und weiter Aerosol erzeugt, das vom Nutzer eingeatmet wird. Erst wenn der Unterdruck kleiner als ein bestimmter Mindestwert ist oder wenn er auf Null gefallen sein sollte, wird die Aerosolerzeugung eingestellt. Der Piezokristall arbeitet dann nur noch als Messgerät zur Erfassung des Druckes im Atemrohr.

Für eine besonders stabile Funktion eines erfindungsgemäßen Inhalators schlägt die Erfindung vor, dass die elektronische Steuerung drei in ihrer Funktion voneinander getrennte Baugruppen aufweist und zwar einen Wechselspannungsgenerator, eine Flusserkennungseinheit und einen Umschalter. Durch diesen Umschalter wird der Wechselspannungsgenerator und die Flusserkennungseinheit wechselweise mit dem Piezokristall elektrisch verbunden. Der Wechselspannungsgenerator gibt Wechselströme ab, deren Spannung und deren Frequenz auf die mechanische Eigenresonanz und die Spannungsempfindlichkeit des Piezokristalls abgestimmt sind und ihn deshalb in resonante Schwingungen versetzen.

Der entscheidende Vorteil der erfindungsgemäßen Doppelnutzung des Piezokristalls ist, dass man ohne zusätzlichen mechanischen Aufwand sondern einfach nur durch die Auswertung der vom Piezokristall erzeugten Spannung auch die Aktorfunktion der Membran ansteuert.

In einer besonderen Ausgestaltung ist der Piezokristall in sich geschlossen und umläuft die Austrittsöffnung damit vollständig, so dass die Membran ausschließlich mit dem Piezokristall verbunden zu werden braucht. Nicht nur bei runder Austrittsöffnung empfiehlt sich die Verwendung eines kreisförmigen Piezokristalls.

Während eines Atemzuges wechselt also die Steuerung mehrfach zwischen dem ersten Betriebszustand, der Druckmessung im Atemrohr und dem zweiten Betriebszustand, der Aerosolerzeugung durch den Piezokristall.

Dabei ist es ein wichtiger Vorteil der erfindungsgemäßen Anordnung, dass dieser Wechsel recht häufig stattfinden kann. Die typischen Werte für die Frequenz zur Ansteuerung des Piezokristalls als Aktor zur Aerosolerzeugung in einer Größenordnung von rund 100 kHz plausibilisieren, wie kurz der Einschwingvorgang ist und wie schnell deshalb der Wechsel vom ersten Betriebszustand als Sensor zum zweiten Betriebszustand als Aktor sein kann. Deshalb schlägt die Erfindung vor, dass der Wechsel zwischen den Betriebszuständen während eines Atemzuges mehrfach, aber vorzugsweise wenigstens 50-mal in der Sekunde erfolgt.

In einer bevorzugten Ausgestaltung erfolgt der Wechsel zwischen den beiden Betriebszuständen entsprechend dem Takt der ebenso mit Hilfe des Piezokristalls erfassten Atemfrequenz. Eine Anpassung an das individuelle Atemvorhalten des Patienten ist durch diese Maßnahme möglich.

Ein weiteres, unerlässliches Merkmal eines erfindungsgemäßen Inhalators ist die Membrane. Sie erzeugt Aerosol, indem sie durch den Piezokristall zu sehr schnellen Schwingungen angeregt wird. Bei diesen Schwingungen wird die federnde Membrane an die Unterseite der Menge an Wirkflüssigkeit gedrückt, die im Wirkflüssigkeitsbehälter steht. Durch diese schnelle Bewegung wird eine winzig kleine Flüssigkeitsmenge in jede Pore hinein gepresst und tritt auf der anderen Seite der Pore jeweils als kleines Flüssigkeitströpfchen wieder aus. Dieses Flüssigkeitströpfchen entspricht in seinen Abmessungen dem kleinsten Durchmesser und der effektiven Länge der Poren. Deshalb schlägt die Erfindung vor, dass der kleinste Durchmesser der überwiegenden Anzahl der Poren in der Membrane kleiner als etwa 200 Mikrometer ist. Die Länge der Poren und damit auch die Stärke der Membrane sollte nicht größer als etwa 10 Mikrometer sein.

Für eine noch effizientere Bildung von noch kleineren Tröpfchen schlägt die Erfindung vor, dass die Poren in ihrem Querschnitt zur Wirkflüssigkeit hin trichterförmig erweitert sind. Mehrere Versuche haben bestätigt, dass ein Öffnungswinkel des Trichters von kleiner/gleich 90° für eine Bildung von besonders kleinen Aerosoltröpfchen sorgt.

Für eine besonders stabile Funktion eines erfindungsgemäßen Inhalators schlägt die Erfindung vor, dass die elektronische Steuerung drei in ihrer Funktion voneinander getrennte Baugruppen aufweist und zwar einen Wechselspannungsgenerator, eine Flusserkennungseinheit und einen Umschalter. Durch diesen Umschalter wird der Wechselspannungsgenerator und die Flusserkennungseinheit wechselweise mit dem Piezokristall elektrisch verbunden. Der Wechselspannungsgenerator gibt Wechselströme ab, deren Spannung und deren Frequenz auf die mechanische Eigenresonanz und die Spannungsempfindlichkeit des Piezokristalls abgestimmt sind und ihn deshalb in resonante Schwingungen versetzen.

Die Flusserkennungseinheit enthält ein Spannungsmessgerät, das den aktuellen Wert der Spannung erfasst, die vom Piezokristall abgegeben wird, wenn die Membran durch die Atemluft im Atemrohr einem Überdruck beim Einatmen und einem Überdruck beim Ausatmen ausgesetzt ist und dadurch den Piezokristall etwas komprimiert oder etwas extrahiert.

Da dieser Spannungswert deutlich kleiner ist als die Spannung zum Betrieb des Piezokristalls als Aktor, wird dafür ein entsprechender Verstärker benötigt. Dieser Verstärker muss idealerweise eine lineare Charakteristik haben oder zumindest eine entsprechende Kompensationsschaltung.

Der Umschalter wird in der Praxis zumeist ein Halbleiter sein, der eine ausreichend hohe Potentialtrennung zwischen der hohen Spannung für den Aktorbetrieb und der niedrigen Spannung für den Sensorbetrieb gewährleistet.

In das Flusserkennungsgerät ist neben dem Spannungsmessgerät, das als erstes die Spannung des Piezokristalls in seiner Sensorfunktion aufnimmt, auch noch eine Bewertungseinheit eingebaut. Sie nimmt den vom Spannungsmessgerät ausgegebenen Wert auf und bewertet auch seine Polarität und unterscheidet im Ausgang nur die drei Stufen "Einatmen" und "Ausatmen" und "keine Atembewegung". Sie gibt also an die Steuerung nur aus, welche der drei Zustände derzeit herrschen.

In einer weiter verfeinerten Ausführungsform dieser Bewertungseinheit ist es sinnvoll, für die Unterscheidung zwischen diesen drei Stufen verschiedene Pegel der Spannungen einstellen zu können, mit denen fein abgeglichen werden kann, ob sich bei diesem Druckwert im Atemrohr eine Erzeugung von Aerosol noch oder schon als sinnvoll erweist.

Eine weitere, sinnvolle aber optionale Baugruppe in der Flusserkennungseinheit ist eine Speicherbaugruppe. Sie speichert den Verlauf der Spannungswerte über die Zeit hinweg. Dadurch kann sie einen mittleren Wert für die Dauer eines typischen Atemzuges ermitteln. Auf dieser Basis kann sie eine weitere Meldung an die Steuerung abgeben, nämlich dass der wesentliche Teil - z.B. 80 % - der typischen Einatmungszeit bereits verstrichen ist.

Aufgrund dieser Meldung wird von der Steuerung bereits kurz vor Ende der Einatmung die Aerosolproduktion eingestellt und damit haben bestätigt, dass ein Öffnungswinkel des Trichters von kleiner/gleich 90° für eine Bildung von besonders kleinen Aerosoltröpfchen sorgt.

Die Flusserkennungseinheit enthält ein Spannungsmessgerät, das den aktuellen Wert der Spannung erfasst, die vom Piezokristall abgegeben wird, wenn die Membran durch die Atemluft im Atemrohr einem Überdruck beim Einatmen und einem Überdruck beim Ausatmen ausgesetzt ist und dadurch den Piezokristall etwas komprimiert oder etwas extrahiert.

Da dieser Spannungswert deutlich kleiner ist als die Spannung zum Betrieb des Piezokristalls als Aktor, wird dafür ein entsprechender Verstärker benötigt. Dieser Verstärker muss idealerweise eine lineare Charakteristik haben oder zumindest eine entsprechende Kompensationsschaltung.

Der Umschalter wird in der Praxis zumeist ein Halbleiter sein, der eine ausreichend hohe Potentialtrennung zwischen der hohen Spankristall 4 und einer Membrane 5, die die Wirkflüssigkeit 1 von der Atemluft 8 trennt, die im Atemrohr 6 strömt, mit dem die Membrane 5 durch den Rohrstutzen 6 verbunden ist.

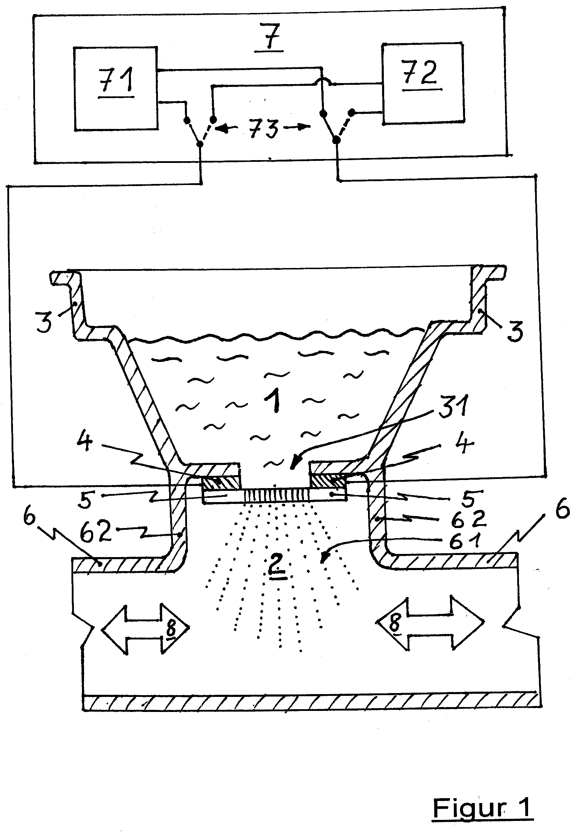

In Figur 1 ist das Atemrohr 6 zeichnerisch in seiner Längsrichtung geschnitten. Die Schnittebene ist auch in die Mitte des Rohrstutzens 62 gelegt, der den Wirkflüssigkeitsbehälter 3 mit dem Atemrohr 6 verbindet. Darüber ist als Blockschema die elektronische Steuerung 7 zur Auswertung und Ansteuerung des Piezokristalls 4 mit ihren wesentlichen drei Baugruppen 71 - 73 dargestellt. In Bildmitte ist der Schnitt durch den Wirkflüssigkeitsbehälter 3 sichtbar, der mit dem Wirkstoff 1 in flüssiger Lösung befüllt ist. Dabei ist in aller Regel das Lösungsmittel für den Wirkstoff reines Wasser.

In die Unterseite des Wirkflüssigkeitsbehälters 3 ist die Austrittsöffnung 31 eingebracht. Dadurch kann der Wirkstoff 1 den Wirkflüssigkeitsbehälter 3 nach unten hin verlassen. Er gelangt dann in die Öffnung des ringförmigen Piezokristalls 4, der an der Unterseite des Wirkflüssigkeitsbehälters 3 befestigt ist. Von der Ringform des Piezokristalls 4 sind in der senkrecht zur Ebene des Ringes verlaufenden Schnittebene nur die beiden rechteckigen schraffierten Schnittflächen zu sehen.

Auf der nach unten weisenden Fläche des Piezokristalls 4 ist die Membran 5 angebracht. Sie ist in der schematischen Figur 1 der Erkennbarkeit halber erheblich dicker gezeichnet, als sie es in der Praxis mit einer Stärke von höchstens 100 Mikrometer wäre. Dank dieser etwas verzerrten Darstellung ist die Membran 5 jedoch deutlich zu erkennen.

Die zahlreichen, sehr kleinen Poren, die die Membrane durchlöchern sind in Figur 1 durch vertikale Striche angedeutet. Dadurch wird in Figur 1 sehr gut nachvollziehbar, auf welchem Weg der Wirkstoff 1 oberhalb der Membran 5 zu einem Aerosol 2 unterhalb der Membran 5 umgewandelt wird.

Zu diesem Zweck ist der Piezokristall 4 elektrisch mit der elektronischen Steuerung 7 verbunden. Diese elektrische Verbindung ist in Figur 1 durch je ein Kabel dargestellt, das sich zu beiden Seiten des Wirkflüssigkeitsbehälters 3 jeweils vom Piezokristall 4 bis zur Steuerung 7 erstreckt.

In der Steuerung 7 ist der Umschalter 73 zur Umschaltung des Piezokristalls 4 von der Wechselspannungsquelle 71 auf die Flusserkennungseinheit 72 eingezeichnet und zwar symbolisch als ein zweipoliger Umschalter mit mechanischen Kontakten. In der Praxis wird jedoch wohl stets ein Halbleiter als Umschalter genutzt werden.

Für die zuvor angesprochene Aktivierung des Piezokristalls 4 zur Erzeugung von Aerosol 2 wird der Umschalter 73 in die links dargestellte Position geschaltet, in welcher er den Piezokristall 4 mit der Wechselspannungsquelle 71 verbindet. Sie erzeugt in der Praxis eine Spannung von z.B. 60 Volt mit einer Frequenz von über 100 kHz. Durch das Anlegen dieser Spannung gerät der Piezokristall 4 in Schwingungen, vorrangig in Richtung seiner Längsachse, in Figur 1 senkrecht.

Durch diese Schwingungen wird auch die Membrane 5 bewegt und zwar senkrecht zu ihren beiden großen Flächen. In Figur 1 ist nachvollziehbar, wie sie sich dadurch etwas in den flüssigen Wirkstoff 1 hineindrückt. Dabei wird eine sehr kleine Teilmenge des Wirkstoffes 1 in jede der feinen Poren der Membrane 5 gedrückt und tritt auf der anderen Seite der Poren an der Unterseite der Membrane 5 als sehr feines Tröpfchen wieder aus.

Dort bildet es zusammen mit den sehr zahlreichen anderen feinen Tröpfchen der Wirkflüssigkeit 1 durch Vermischen mit der Atemluft 8 das Aerosol 2, das in Figur 1 durch Punkte dargestellt ist. Die Größe und die Anzahl dieser Punkte sind jedoch keine maßstäbliche Darstellung der tatsächlich existierenden Aerosoltröpfchen. In der Praxis ist die Anzahl ganz erheblich höher und der Durchmesser der Größenordnungen kleiner.

Figur 1 zeigt, wie das Aerosol 2 durch den Rohrstutzen 62 und die Aerosolöffnung 61 in das Atemrohr 6 eintritt. Dort strömt die Atemluft 8 und vermischt sich mit dem Aerosol 2. Figur 1 verdeutlicht, dass das Gemisch aus Atemluft 8 und Aerosol 2 sich in beide Richtungen gleichmäßig bewegen kann, was durch große Doppelpfeile symbolisiert ist.

Dadurch wird sofort verständlich, dass das Aerosol 2 nur dann produziert werden sollte, wenn die Atemluft 8 eingeatmet wird, nicht aber wenn die Atemluft 8 ausgeatmet wird. Nur dann gelangt der Wirkstoff 1 vollständig in die Lungen und wird nicht beim Ausatmen aus dem Atemrohr 6 zusammen mit der Atemluft 8 heraus geblasen. Weil dieser Zusammenhang auch ohne besondere grafische Darstellung sofort nachvollziehbar ist, ist er in Figur 1 nicht eingetragen.

Figur 1 ist auch hilfreich, um den Betriebszustand "Druckmessung im Atemrohr 6" nachzuvollziehen. Das Ausatmen unterscheidet sich vom Einatmen nicht nur durch die Richtung der Luftströmung, sondern auch durch den Druck im Atemrohr 6: Er ist beim Ausatmen höher als der atmosphärische Luftdruck und beim Einatmen niedriger, es herrscht also ein Unterdruck. Dieser Druck wirkt auch auf die Membrane 5. Sie wird beim Ausatmen auf den Piezokristall 4 gedrückt und komprimiert ihn dadurch, woraufhin er eine elektrische Spannung an seinen Anschlüssen erzeugt.

Beim Einatmen wird die Membrane 5 durch den Unterdruck ebenfalls bewegt und zwar in einer vom Piezokristall 4 abgewandten Richtung, wodurch er etwas gelängt wird und daraufhin ebenfalls eine elektrische Spannung an seinen Anschlüssen erzeugt; jedoch mit anderer Polarität.

Sowohl beim Ein- als auch beim Ausatmen muss zur Druckmessung in der elektronischen Steuerung 7 der Umschalter 73 den Piezokristall 4 elektrisch mit der Flusserkennungseinheit 72 verbinden. Das ist die in Figur 1 gestrichelt dargestellte Stellung der beiden symbolischen Kontakte.

In Figur 1 ist die Funktion der Flusserkennungseinheit 72 nicht weiter ausgeführt. Deshalb ist auch nicht dargestellt, dass sie eine von den drei Meldungen "Einatmen" oder "Ausatmen" oder "Atemstillstand" erzeugt und an die Steuerung 7 zu einer - in Figur 1 ebenfalls nicht dargestellten - Baugruppe weiterleitet, die dann für die Ansteuerung des Umschalters 73 verantwortlich ist und sicherstellt, dass nur während des Einatmens Aerosol 2 erzeugt wird.

In Figur 1 ist gut erkennbar, dass der erfindungsgemäße Inhalator im Vergleich zu den Inhalatoren auf dem bekannten Stand der Technik nur eine sehr geringe Anzahl an verschiedenen, mechanischen Bauteilen aufweist und mit einem Minimum an Bauraum und insbesondere wenigen zu reinigenden Oberflächen auskommt.

### Bezugszeichenliste

- 1: Wirkstoff, in Flüssigkeit gelöst
- 2: Aerosol aus Wirkstofftröpfchen und Luft
- 3: Wirkflüssigkeitsbehälter
- 31: Austrittsöffnung an Unterseite des Wirkflüssigkeitsbehälters 3
- 4: Piezokristall, umgibt Austrittsöffnung 31
- 5: Membran, auf Unterseite des Piezokristalls 4
- 6: Atemrohr, über Rohrstutzen 62 mit Membran 5 verbunden
- 61: Aerosolöffnung in Atemrohr 6
- 62: Rohrstutzen zur Verbindung der Membran 5 mit der Aerosolöffnung 61
- 7: elektronische Steuerung zur Auswertung und Ansteuerung des Piezokristalls 4
- 71: Wechselspannungsquelle in Steuerung 7
- 72: Flusserkennungseinheit in Steuerung 7
- 73: Umschalter in Steuerung 7 zur Umschaltung des Piezokristalls 4 von Wechselspannungsquelle 71 auf Flusserkennungs-Einheit 72
- 8: Atemluft, strömt in Atemrohr 6

## Patentansprüche

1. Inhalator zum Inhalieren eines Wirkstoffes (1) in Form eines Aerosols (2) in die Lungen eines Lebewesens, bestehend aus
- einem Wirkflüssigkeitsbehälter (3), in dessen Unterseite eine Austrittsöffnung (31) eingebracht ist und
- einem Piezokristall (4),
- der an der Unterseite des Wirkflüssigkeitsbehälters (31) angebracht ist und
- der die Austrittsöffnung (31) zumindest stückweise umschließt
und
- einer Membran (5),
- die von zahlreichen Poren durchbrochen ist und
- die auf der Unterseite des Piezokristalls (4) aufliegt und
- die die Austrittsöffnung (31) verschließt und
- einem Atemrohr (6),
- durch das Atemluft in die Lungen eingesaugt und wieder ausgestoßen wird und
- das von einer Aerosolöffnung (61) durchbrochen ist, welche sich an der Längsseite des Atemrohres (6) befindet
und direkt oder über einen Rohrstutzen (62) von der Unterseite der Membran (5) verschlossen wird und
- einer elektronischen Steuerung (7), die mit dem Piezokristall (4) elektrisch verbunden ist,
wobei ein Sensor vorhanden ist und von der Steuerung (7)
- in einem ersten Betriebszustand zwecks Druckmessung im Atemrohr (6)
- eine vom Sensor abgegebene Spannung erfasst wird und
- aus diesem Signal abgeleitet wird, ob die Atemluft im Atemrohr (6) eingeatmet oder ausgeatmet wird oder stillsteht und
- in einem zweiten Betriebszustand zwecks Aerosolerzeugung der Piezokristall (4) nur während des Einatmens mit einer hochfrequenten Wechselspannungsquelle (71) verbunden wird,
**dadurch gekennzeichnet, dass**
- der Sensor der Piezokristall (4) ist und,
- die Steuerung (7) einen Wechselspannungsgenerator (71) und eine Flusserkennungseinheit (72) beinhaltet, die durch einen Umschalter (73) wechselweise mit dem Piezokristall (4) elektrisch verbunden werden,
- wobei die Spannung und die Frequenz des Wechselspannungsgenerators (71) den Piezokristall (4) in resonante Schwingungen versetzt, und
- wobei die Flusserkennungseinheit (72) mit einem Spannungsmessgerät
- den aktuellen Wert der Spannung erfasst, die vom Piezokristall (4) abgegeben wird, wenn er über die Membran (5) hinweg durch die Atemluft im Atemrohr (6) einem Unterdruck beim Einatmen und einem Überdruck beim Ausatmen ausgesetzt ist und in einer
- Bewertungseinheit die Polarität und den Wert der Spannung auswertet und abhängig davon eine der drei Meldungen "Einatmen" oder "Ausatmen" oder "Keine Atembewegung" an die Steuerung (7) weitergibt.

2. Inhalator nach Anspruch 1, **dadurch gekennzeichnet, dass** der Piezokristall (4) in sich geschlossen, insbesondere kreisförmig ist.

3. Inhalator nach Anspruch 1, **dadurch gekennzeichnet, dass** die Steuerung (7) während eines Atemzuges des mit dem Atemrohr (6) verbundenen Lebewesens mehrfach zwischen dem ersten Betriebszustand und dem zweiten Betriebszustand wechselt.

4. Inhalator nach einem der vorhergehenden Ansprüche, **dadurch gekennzeichnet, dass** der kleinste Durchmesser der Poren in der Membrane (5) kleiner als etwa 10 Mikrometer ist.

5. Inhalator nach einem der vorhergehenden Ansprüche, **dadurch gekennzeichnet, dass** die Stärke der Membrane (5) kleiner als etwa 100 Mikrometer ist.

6. Inhalator nach einem der vorhergehenden Ansprüche, **dadurch gekennzeichnet, dass** die Poren zur Wirkflüssigkeit hin trichterförmig erweitert sind, wobei der Trichter vorzugsweise einen Öffnungswinkel von kleiner/gleich 90° aufweist.

7. Inhalator nach einem der vorhergehenden Ansprüche, **dadurch gekennzeichnet, dass** die Umschaltung zwischen Flusserkennungseinheit (72) und Wechselspannungsgenerator (71) mindestens 50 mal in der Sekunde erfolgt.

8. Inhalator nach einem der vorhergehenden Ansprüche, **dadurch gekennzeichnet, dass** in die Flusserkennungseinheit (72) eine Speicherbaugruppe eingefügt ist, in der der Verlauf der Spannungswerte über die Zeit hinweg speicherbar ist und die einen mittleren Wert für die Dauer eines Atemzuges ermittelt und die eine Meldung an die Steuerung (7) abgibt, wenn 80% oder ein anderer Anteil der mittleren Einatmungszeit verstrichen ist.

9. Inhalator nach Anspruch 8, **dadurch gekennzeichnet, dass** die Steuerung (7) dazu eingerichtet ist
- die Erzeugung von Aerosol (2) zu aktivieren, sobald der gemessene Druck im Atemrohr (6) einen bestimmten Wert unterschreitet und
- die Erzeugung von Aerosol (2) zu beenden, sobald etwa 80% oder ein anderer, relativ großer Anteil der zuvor gemessenen mittleren Einatmungsdauer erreicht ist.

## Claims

1. Inhaler for inhaling an active substance (1) in the form of an aerosol (2) into the lungs of a living organism, consisting of
- an active liquid vessel (3), in the underside of which an outlet opening (31) is introduced, and
- a piezo crystal (4),
- which is mounted on the underside of the active liquid vessel (31), and
- which, at least piecewise, surrounds the outlet opening (31) and
- a membrane (5),
- which is penetrated by a multiplicity of pores and
- which rests on the underside of the piezo-crystal (4) and
- which seals the outlet opening (31) and
- a breathing tube (6),
- through which the respiratory air is sucked into the lungs and expelled again and
- which is penetrated by an aerosol opening (61),
- which is located on the longitudinal side of the respiratory tube (6) and is sealed directly or via a pipe socket (62) from the underside of the membrane (5) and
- an electronic control means (7), which is electrically connected to the piezo crystal (4),
a sensor being present and, by the control means (7),
- in a first operating state, for the purpose of pressure measurement in the respiratory tube (6)
- a voltage emitted by the sensor being registered and
- from this signal it being derived whether the respiratory air in the respiratory tube (6) is inhaled or exhaled or is stationary
and
- in a second operating state, for the purpose of aerosol generation, the piezo crystal (4) is only connected to a high-frequency alternating voltage source (71) during inhalation, **characterised in that**
- the sensor is the piezo crystal (4) and
- the control means (7) includes an a.c. voltage generator (71) and a flow identification unit (72), which, by means of a changeover switch (73), are alternately electrically connected to the piezo crystal (4),
- the voltage and the frequency of the alternating voltage generator (71) setting the piezo crystal (4) into resonant oscillation, and
- the flow identification unit (72), by means of a voltage meter,
- registering the current value of the voltage, which is output by the piezo crystal (4) when, across the membrane (5), via the respiratory air in the respiratory tube (6), it is subjected to a subatmospheric pressure during inhalation and a superatmospheric pressure during exhalation, and in an
- evaluation unit, the polarity and the value of the voltage are evaluated and, dependent thereon, transmits one of the three messages "inhalation" or "exhalation" or "no respiratory movement" to the control means (7).

2. Inhaler according to claim 1, **characterised in that** the piezo crystal (4) is self-contained, in particular circular.

3. Inhaler according to claim 1, **characterised in that** during a respiration of the living organism connected to the respiratory tube (6), the control means (7) changes multiply between the first operating state and the second operating state.

4. Inhaler according to one of the preceding claims, **characterised in that** the smallest diameter of the pores in the membrane (5) is smaller than about 10 micrometers.

5. Inhaler according to one of the preceding claims, **characterised in that** the thickness of the membrane (5) is smaller than about 100 micrometers.

6. Inhaler according to one of the preceding claims, **characterised in that** the pores are enlarged in the manner of a funnel towards the active liquid, the funnel preferably having an opening angle of less than/equal to 90°.

7. Inhaler according to one of the preceding claims, **characterised in that** the changeover between the flow identification unit (72) and an alternating voltage generator (71) takes place at least 50 times per second.

8. Inhaler according to claim 8, **characterised in that**, into the flow identification unit (72), a storage module is inserted, in which the variation of the voltage values over time can be stored, and which determines an average value for the duration of a breath and outputs a message to the control means (7) when 80% or another proportion of the average inhalation time has passed.

9. Method for actuating an inhaler according to claim 9, **characterised in that**
- the generation of aerosol (2) is activated as soon as the measured pressure in the respiration tube (6) falls below a particular value and
- the generation of aerosol (2) is terminated as soon as about 80% or another, relatively large proportion of the previously measured mean inhalation duration has been reached.

## Revendications

1. Inhalateur destiné à inhaler un agent actif (1) sous la forme d'un aérosol (2) dans les poumons d'un être vivant, consistant en
- un réservoir de liquide actif (3) sur la face inférieure duquel a été pratiquée une ouverture de sortie (31)
- un cristal piézométrique (4)
- qui a été placé sur la face inférieure du réservoir de liquide actif (31) et
- qui entoure l'ouverture de sortie (31) au moins en partie
et
- une membrane (5),
- qui est percée de nombreux pores,
- qui repose sur la face inférieure du cristal piézométrique (4) et
- qui referme l'ouverture de sortie (31) et
- un tube respiratoire (6)
- à travers lequel l'air respirable est inspiré dans les poumons et ensuite à nouveau expiré hors de ces derniers et
- qui est percé d'une ouverture pour aérosol (61) qui se trouve sur le côté longitudinal du tube respiratoire (6) et est refermée, directement ou par l'intermédiaire d'un embout de tube (62), par la partie inférieure de la membrane (5) et
- une commande électronique (7) qui est reliée électriquement au cristal piézométrique (4),
sachant qu'il existe un capteur, et que la commande (7)
- dans premier un état de service visant à mesurer la pression dans le tube respiratoire (6)
- enregistre une tension rendue par le capteur et
- qu'il est dérivé de ce signal si l'air respirable est inspiré dans le tube respiratoire (6) ou expiré dans ce dernier, ou bien est arrêté
et
- dans un second état de service visant à générer l'aérosol, le cristal piézométrique (4) est relié à une source de tension alternative haute fréquence (71) seulement pendant la respiration
**caractérisé par le fait que**
- le capteur est le cristal piézométrique (4) et que
- la commande (7) comprend un générateur de tension alternative (71) et une unité de détection de flux (72) qui sont alternativement reliés électriquement au cristal piézométrique (4) par un commutateur (73),
- sachant que la tension et la fréquence du générateur de tension alternative (71) met le cristal piézométrique (4) en vibrations résonnantes et
- sachant que l'unité de détection de flux (72), à l'aide d'un appareil de mesure de la tension,
- enregistre la valeur actuelle de la tension qui est émise par le cristal piézométrique (4) lorsque ce dernier est, à travers la membrane (5) et par l'air respirable dans le tube respiratoire (6), exposé à une sous-pression lors de l'inspiration et à une surpression lors de l'expiration et
- évalue dans une unité d'évaluation la polarité et la valeur de la tension, et transmet en fonction de ces dernières un des trois messages « inspirer », « expirer » ou « pas de mouvement de respiration » à la commande (7).

2. Inhalateur selon la revendication 1, **caractérisé par le fait que** le cristal piézométrique (4) est refermé sur lui-même, notamment de forme circulaire.

3. Inhalateur selon la revendication 1, **caractérisé par le fait que** la commande (7) change plusieurs fois entre le premier état de service et le second état de service pendant une respiration de l'être vivant relié au tube respiratoire (6).

4. Inhalateur selon une des revendications précédentes, **caractérisé par le fait que** le plus petit diamètre des pores dans la membrane (5) est inférieur à environ 10 micromètres.

5. Inhalateur selon une des revendications précédentes, **caractérisé par le fait que** l'épaisseur de la membrane (5) est inférieure à environ 100 micromètres.

6. Inhalateur selon une des revendications précédentes, **caractérisée par le fait que** les pores sont élargis en forme d'entonnoir vers le liquide actif, sachant que l'entonnoir présente de préférence un angle d'ouverture inférieur ou égal à 90°.

7. Inhalateur selon une des revendications précédentes, **caractérisée par le fait que** la commutation entre l'unité de détection de flux (72) et le générateur de tension alternative (71) a lieu au moins 50 fois par seconde.

8. Inhalateur selon la revendication 8, **caractérisé par le fait qu'**un sous-ensemble de mémoire est inséré dans l'unité de détection du flux (72), dans lequel le déroulement des valeurs de tension peut être mémorisé au cours du temps et qui détermine une valeur moyenne pour la durée d'une respiration et remet un message à la commande (7) lorsque 80 % ou une autre proportion du temps de respiration moyen est passé.

9. Procédé destiné à commander un inhalateur selon la revendication 9, **caractérisé par le fait que**
- la génération d'aérosol (2) est activée dès que la pression mesurée dans le tube respiratoire (6) passe en dessous d'une certaine valeur (6) et
- la génération d'aérosol (2) est terminée dès qu'environ 80 % ou une autre proportion relativement grande de la durée d'inspiration moyenne préalablement mesurée a été atteinte.
